(19)

**Europäisches Patentamt**
**European Patent Office**
**Office européen des brevets**

(11) **EP 1 847 281 A1**

(12) **EUROPEAN PATENT APPLICATION**

(43) Date of publication:
24.10.2007 Bulletin 2007/43

(51) Int Cl.:
*A61M 1/12* (2006.01)        *A61M 1/10* (2006.01)

(21) Application number: 07251636.2

(22) Date of filing: 18.04.2007

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: 20.04.2006  AU 2006902067
20.04.2006  AU 2006902066

(71) Applicants:
• **Ventrassist Pty Ltd**
**Chatswood, NSW 2067 (AU)**
• **THE UNIVERSITY OF NEW SOUTH WALES**
**Kensington, NSW 2052 (AU)**

(72) Inventors:
• **Ayre, Peter Joseph**
**Crows Nest NSW 2065 (AU)**
• **Mason, David**
**Balwyn North VIC 3104 (AU)**
• **Karantonis, Dean Michael**
**Kensington NWS 2033 (AU)**

(74) Representative: **Beresford, Keith Denis Lewis**
**Beresford & Co.,**
**16 High Holborn**
**London WC1V 6BX (GB)**

(54) **System and method of controlling a rotary blood pump**

(57)    A method of and apparatus for controlling the speed of a rotary blood pump, which comprises the measuring the speed and/or power of said pump, calculating an array of system parameters derived from the measured speed, analysing these parameters, and if the analysis indicates ventricular collapse or imminent ventricular collapse, then the speed of said pump is altered, to minimise the risk of the collapse occurring. Preferably the analysis is done using a Classification and Regression Tree (CART) analysis.

Fig. 1

**EP 1 847 281 A1**

**Description**

**Field of the Invention**

The present invention relates to improvements in systems to minimise or avoid ventricular collapse in patients implanted with a blood pump.

**Background of the Invention**

Blood pumps have been commonly used to provide mechanical support or assistance to the left ventricles of patients. Typically, the left ventricle of the heart is responsible for pumping blood into the aorta and throughout a majority of the patient's body. Ventricular assistance can be provided by implanted blood pumps, such as the VentrAssist™ rotary blood pump described in US Patent 6,227,797 (Watterson et al).

These blood pumps generally pump blood in parallel to the normal circulatory system by removing blood directly from the left ventricle and pumping into a portion of the aorta. Generally when such a blood pump is implanted, blood may flow or be pumped by both the left ventricle and the blood pump.

The speed of the implanted blood pump is carefully monitored and controlled. Preferably, the pump and the respective controller should be able to adapt to changes in physiological demand for blood of the patient's body. Preferably, the blood pump should not be allowed to run so fast the pump causes a suction event whereby the pump receives less blood flow and the contractile properties of the ventricle are effectively lost or diminished. In severe situations of a suction event, the ventricle wall is pulled over an inflow of the blood pump and may completely occlude blood flow throughout the left ventricle and the blood pump.

In the past, if an implanted blood pump pumps blood at a too high rate, when compared to the left ventricle, the heart may experience arrhythmias. Additionally, if the pump is operating at a relative speed that is too low, when compared to the left ventricle, the patient may experience pulmonary oedemas.

US Patent 6,949,066(Bearnson et al) discloses a pump control system for use with a centrifugal type blood pump of the kind used as left ventricle assist devices. The system includes a first sensor that detects at least one operational parameter of the pump; and a second sensor that detects and measures at least one physiological parameter of a patient implanted with the pump. This system fails to address situations wherein intermittent suction events are occurring to a patient implanted with a left ventricle assist device. Additionally, the addition of sensors to the system will add to its complexity; increase the likelihood of device failure and reduce biocompatibility.

US Patent 6,991,595 (Burke et al) describes an adaptive speed control for an implanted blood pump wherein the control is adapted to detect the onset of left ventricular collapse by deriving and monitoring a pulsatility index, and adjusting the pump speed to maintain the pulsatility index at a pump set-point. The pulsatility index pump set-point is decreased incrementally when the onset of ventricular collapse has not been detected for predetermined period of time. Experimentally, it has been found that pulsatility index is not the most preferred physiological characteristic for determining the imminence of a suction event in isolation due to inaccuracies and unreliability of pulsatility indices.

US Patent 6,783,328 (Lucke et al) discloses a pump control system that monitors flow and/or pressure of the pump output and decides whether the flow or pressure exceeds a critical level. If the critical level is exceeded, the control system reduces the pumping speed by reducing the pumping speed set-point. This system relies on the expectation that the all of the pressure and/or flow experienced by blood at the pump outlet is the solely the output of the pump, this system ignores other pumping elements such as the natural heart which is still capable of supplying a proportion of flow and pressure. The system also only detects whether the threshold flow or pressure values have been exceeded and does not detect or determine any suction events.

US Patent 5,888,242 (Antaki) discloses an automatic speed control system for implanted blood pump wherein the speed of the pump is incrementally increased and when the system detects the imminence of a suction event occurring to the left ventricle, the system decreases the pump speed by a predetermined amount. A disadvantage with this system is that when the system detects the imminence of a suction event, the system slows the pump and then gradually increases the speed until the imminence is detected again. Hence the system continually repeats the error despite its detection and this may be dangerous for the patient.

US Patent 6,066,086 (Antaki) discloses a further automatic speed control system for use with an implanted cardiac assist blood pump wherein the system operates in a manner to prevent the opening of the aortic valve during the operation of the heart, once it has been implanted with a left ventricle assist device. Experimentally, the inventors of the present specification have found that is preferable to allow the aortic valve to open and close during the operation of a left ventricular assist device. This disclosure does not measure or predict suction events.

US Patent 6,623,420 (Reich et al) discloses a system wherein an implanted blood pump is connected to a single blood pressure sensor which is positioned in the inflow of the pump. The sensor continuously detects blood pressures within the cannula and compares the detected blood pressure to a tabulated predicted blood pressure. The system then adjusts the pump speed to minimise the difference between the detected value and the predicted value. Suction events are not directly avoided by this system and the inclusion of pressure sensors in the blood flow path is generally not preferred for patient safety reasons.

US Patent 6,443,983 (Nagyszalancy et al) discloses a pump speed control system wherein blood flow and pressure are both detected and measured and then used in a feedback to control speeds of two blood pumps. The system requires measured values for both flow and pressure to function and as such the system requires the additional implantation or use of sensors in the blood path, which is preferably avoided as they may lead to points of blood flow stagnation or blood clotting.

PCT International Publication No. WO 03/057013 (Morello) describes a further control system wherein the system includes a feedback from an implanted flow sensor. As previously stated, the use of implanted sensors should preferably be avoided or minimised. Furthermore, the system generates a suction probability index and this probability index inherently infers a chance of errors or unreliability. Additionally, the lack of certainty as to whether ventricular collapse has occurred should be avoided.

PCT International Publication No. WO 04/028593 (Ayre) describes a further control system for a rotary blood pump. However, this system generally includes a type of sensor and generally does not detect the imminence of ventricular collapse.

The identification of pumping states in implantable rotary blood pumps has also received some attention in the scientific literature. Despite the use of implanted sensors by various research groups, it is recognised that their use should be avoided due to cost and reliability issues. Other efforts by a number of investigators to identify pumping states have focused on waveform analysis of the pump motor feedback signals (electric current or rotor speed), with a range of indices having been derived from these signals as indicators of either over or under-pumping. Despite some success in deriving such indices, this research has until now generally failed to deliver on the key challenge, to develop robust automated algorithms for detection of physiologically significant pumping states.

The present invention aims to provide an alternative, or to address or ameliorate one or more of the disadvantages associated with the abovementioned prior art.

**Brief Description of the Invention**

A first aspect of the present invention involves a control system for controlling the target speed of a rotary blood pump, the control system being adapted to measure the speed and/or power of the pump, and then to calculate at least two system parameters derived from the speed and/or power measurement, and to analyse the parameters, and if the analysis indicate ventricular collapse or imminent ventricular collapse, then the control system alters the target speed of the pump to minimise the risk of the collapse occurring..

Preferably, the system parameters are selected from among a calculation of: the pulsatility index ($N_{pp}$), the change in the pulsatility index ($\Delta N_{pp}$), the second derivative of the speed ($\Delta^2 N$), the $N_{profile}$, the change in $N_{profile}$ ($\Delta N_{profile}$), the change in $N_{max}$ ($\Delta N_{max}$), or the change in $N_{freq}$($\Delta N_{freq}$); these parameters are defined below. A method of controlling the pumping speed of a rotary blood pump is also provided. It is also preferred that the system may alter the target speed by reducing it to a predetermined target speed. The system may preferably use Classification and Regression Tree (CART) analysis to analyse the parameters. Ideally, the pump is an implantable pump.

Another aspect of the invention involves control system for controlling the target speed of a rotary blood pump, the control system being adapted to measure the speed and/or power of the pump, and then to calculate one or more system parameters derived from the speed and/or power measurement, and then to analyse the parameters, using a Classification and Regression Tree (CART) analysis, and if the analysis indicates ventricular collapse or imminence of ventricular collapse, then the control system alters the target speed of the pump to minimise the risk of the collapse occurring. Preferably the system parameters are selected from those mentioned above.

Additionally, the invention concerns apparatus for controlling the target speed of a rotary blood pump, the apparatus having means to measure the speed and/or power of the pump, and means to calculate at least two system parameters derived from the speed and/or power measurement, and means to analyse the parameters, and if the analysis indicates ventricular collapse or imminent ventricular collapse, then the apparatus alters the target speed of the pump to minimise the risk of the collapse occurring. Preferably the system parameters are selected from those mentioned above.

A further embodiment concerns a method of controlling the target speed of a rotary blood pump, the method comprising, measuring of the speed and/or power of the pump, calculating at least two system parameters derived from the speed and/or power measurement, analysing the parameters, and if the analysis indicates ventricular collapse or imminent ventricular collapse, then altering the target speed of the pump so as to minimise the risk of the collapse occurring. Preferably the system parameters are selected from those mentioned above.

**Brief Description of the Drawings**

Embodiments of the present invention will now be described with reference to the accompanying drawings wherein:

Fig. 1 depicts a schematic view of a first embodiment of the present invention;
Fig. 2 depicts a graphical example of pump speed in a patient not experiencing a suction event;
Fig. 3 depicts a graphical example of pump speed in a patient experiencing early stage suction;
Fig. 4 depicts a graphical example of pump speed in a patient experiencing late stage suction;
Fig. 5 depicts a further graph of the aforementioned examples of the pump speed; and
Fig. 6 depicts an example of pumping speed signal filtering;
Fig. 7 depicts an example of speed signal output of a rotary blood pump;
Fig. 8 depicts an example of a decision tree for use with embodiments of the present invention;
Fig. 9 depicts pull-down menu structure for configuring a preferred classification tree;
Fig. 10 depicts a first preferred example of a Graphical User Interface ('GUI') for use with an embodiment;
Fig. 11 depicts second preferred example of a Graphical User Interface ('GUI') for use with an embodiment;
Fig. 12 depicts an example of measurements and signals for use with further preferred embodiments; and

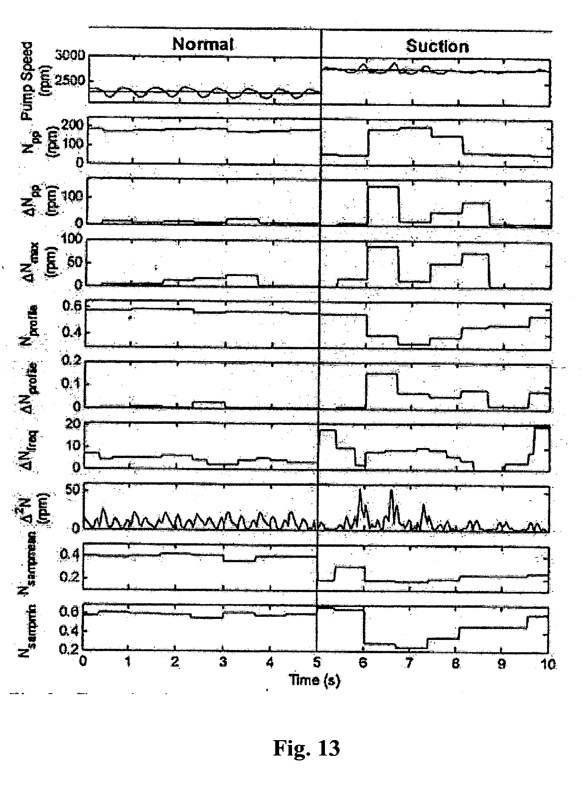

Fig. 13 depicts an example of measurements and signals for use with further preferred embodiments.

**Brief Description of the Preferred Embodiments**

An example of a suitable blood pump for use with the present system is the VentrAssist™ Left Ventricle Assist Device, as described in US Patent 6,227,797 (Watterson et al). This pump includes an impeller with four blades. The four blades each include a permanent magnet which interacts with two sets of stator coils to rotate the impeller, in use. Each set of stator coils are mounted on or in the pump housing on either side of the impeller. The pump also includes a hydrodynamic thrust bearing to suspend the impeller, whilst it is being rotated. In this blood pump, the stator coils and the magnets within the impeller form a brushless DC motor. This brushless DC motor, in this configuration, sends six "back EMF" pulses per full revolution back to the controller, which correlates with the various positions of magnets as they pass the stator coils, when in use. Within the present specification this type of blood pump is used as a preferred example of a suitable such pump, but other pumps may also be utilised, and other such pumps are intended to be within the scope of the present invention.

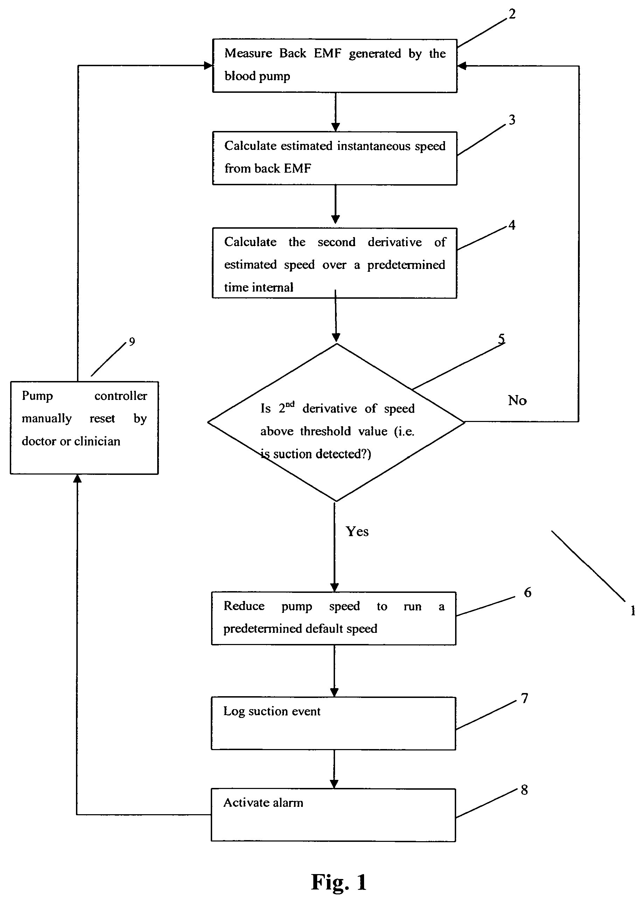

A first embodiment of the present invention is depicted in Fig. 1 of the accompanying drawings. This provides a schematic of a system 1 wherein the system aims to instruct a blood pump to avoid a partial or full suction event.

Generally, the system 1 would be integrated into the design of a controller for use with an implantable mechanical blood pump. Preferably, the blood pump may be a centrifugal type pump capable of being fully implanted within the body of patient. A suitable such pump may be connected in parallel to the normal circulation path and include being connected across the apex of the left ventricle and a portion of the aorta. This connection may be achieved by the use of specialised cannulae.

The system 1 includes several steps or stages whereby the net result may be to avoid or minimise suction events, particularly in connection with the left ventricle. The system of the present invention provides for a better (or alternative) means of controlling the speed of a blood pump. As well as providing good results for avoiding or minimising situations where the pump runs too fast, it may additionally help avoid any build up of blood on the lungs or in other organs, commonly called oedemas, which can be caused by the rotary blood pump operating at a speed that is relatively too low for the physiological state of the patient.

The first step 2 of the system 1 is to measure the speed of the rotary blood pump. This measurement is preferably achieved by measuring the back EMF generated by the impeller of the rotary blood pump as it passes individual stator coils. Preferably, the controller or the system 1 detects this back EMF and may use the detected back EMF to accurately detect and measure the instantaneous speed of the impeller.

Preferably, the system 1 used by the controller then utilises the back EMF signals generated by the brushless DC motor to calculate the instantaneous speed of the impeller. This second step 3 allows the controller to know the speed of the impeller at a particular time and thereby make further calculations based on instantaneous speed.

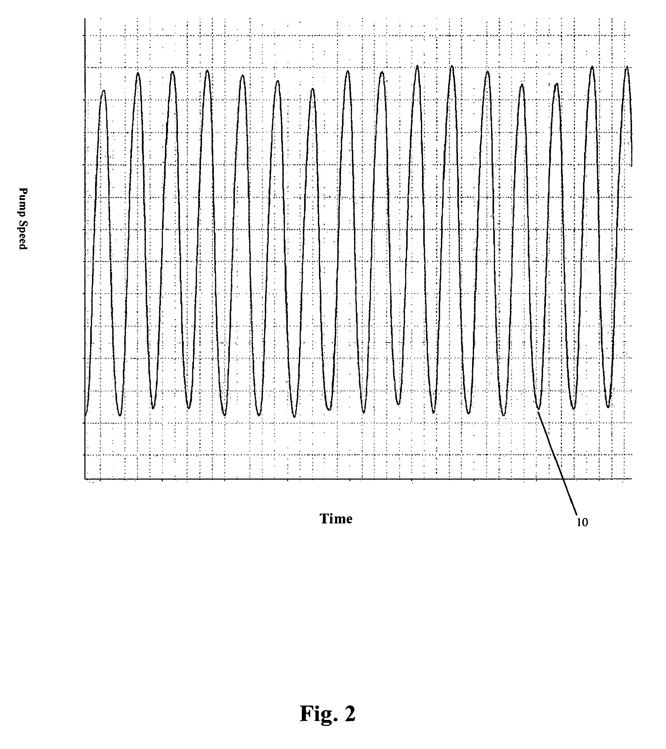

In Fig. 2, instantaneous pump speed is plotted against time in a situation where no suction is occurring. The pump speed fluctuates between an upper and lower limit as the load from the blood increases and decreases over several cardiac cycles. For example, when utilising the VentrAssist™ blood pump, the pump speeds vary generally between an upper limit of 1830 rpm and a lower limit of about 1720 rpm. However, these limits will change and vary depending on the normal pump running speeds and the configuration of the pump. The pump speed in Fig. 2 pulses generally because the left ventricle is still capable of doing work and the septum wall of the left ventricle is not adversely being pulled by the pumping force of the pump. Fig. 2 also represents a pump speed waveform that minimises or avoids ventricular suck down.

The third step 4 according to the example of the system 1 shown in Fig. 1 is for the controller of the rotary blood pump to instantaneously calculate the second derivative of the speed derived in the second step 3. Calculating the second derivative of the instantaneous speed allows the peaks and troughs of the graph depicted in Fig. 2 to be accentuated. The peaks and troughs become more pronounced; sharper and generally lengthier making detection of anomalies easier and more pronounced.

In the fourth step 5 of the system 1, the controller considers whether a suction event is occurring. This is preferably achieved by the system 1 or the controller using the system comparing the calculated second derivative of speed with a threshold value. However, there are other methods described later in this specification. If the calculated second derivative goes above the threshold value, the controller and/or system 1 determines that a suction event has occurred. The second derivative of instantaneous pump speed for a patient not suffering from ventricular suck down is significantly less than the second derivatives of a patient suffering any stage of ventricular suction. The threshold value is preferably determined experimentally and may depend on the rotary blood pump shape and configuration.

When a suction event is not detected by the fourth step 5, the system 1 reverts back to the first step 2 and thereby continuously checks whether suction is occurring each any given time.

The fifth step 6 of the system 1 is activated if suction is detected in the fourth step 5. In this fifth step 6, the controller or the system 1 reduces the pump speed to a safe predetermined default speed. This default speed is preferably determined by the clinician or doctor implanting the rotary blood pump and is set at the time of implantation. Typically, this default speed is slow enough to allow the left ventricle to be contractile whilst maintaining a speed sufficient to prevent blood flow regurgitation through the rotary blood pump. Regurgitation may occur where the pressure generated by the pump is lower than the instantaneous arterial pressure in the aorta. Furthermore, if the pump is completely stopped, blood clotting may occur within the pump inflow and outflow cannulae or within the pump body.

When used in conjunction with a VentrAssist™ blood pump, the system 1 at the fifth step 6 may preferably operate the pump at a speed sufficient to allow the hydrodynamic thrust bearings of the pump to function and this is generally at a speed greater than 1000 rpm. Other shapes and configurations of rotary blood pumps may have differing preferred default speeds.

The sixth step 7 in system 1 is to log the occurrence of the suction event. This generally requires the controller or system 1 to record the time and date of suction event. Also, details of the severity of the suction event may be recorded and these include recordings of the values of instantaneous speed and the specified time. Preferably, the logs of these recorded suction events may be interrogated by doctors, clinicians and staff monitors at a later stage. Data may also be further transmitted or disseminated through networks of computers or into databases.

The seventh step 8 of the system 1 is to activate an alarm on or in the preferred controller. This alarm may be an audible and/or visual alarm. Additionally, the alarm may include a vibrating component to alert the user even when other alarms fail. All of these alarms may alert the user to the suction detection having occurred and thereby instruct the user or patient to contact a clinician or doctor to analyse the situation.

The eighth step 9 in system 1 occurs once the patient has been alerted by seventh step 8 and the patient has visited a doctor or clinician. The doctor or clinician independently analyses the situation that gave rise to the suction event occurring and may take some kind of permanent prevention action and then may manually reset the controller or system 1 to return it back to the first step 2 of the system 1.

Fig. 2 depicts a graphical representation of pump speed plotted against time wherein a typical patient is implanted with a rotary blood pump and the patient is not experiencing full or partial ventricular collapse or a suction event. The line 10 demonstrates the speed fluctuations of the pump as the heart pumps in parallel to the rotary blood pump. The pulsatile nature of the natural patient's heart beating increases and decreases the load acting on the rotary blood pump throughout the cardiac cycle including systole and diastole. The changes in load experienced by the pump affect the pumping speed of the blood pump, which is preferably a centrifugal type apparatus. For example, the pumping speed using the Ventrassist™ blood pump generally fluctuates between 1700-1900 rpm during this normal operation.

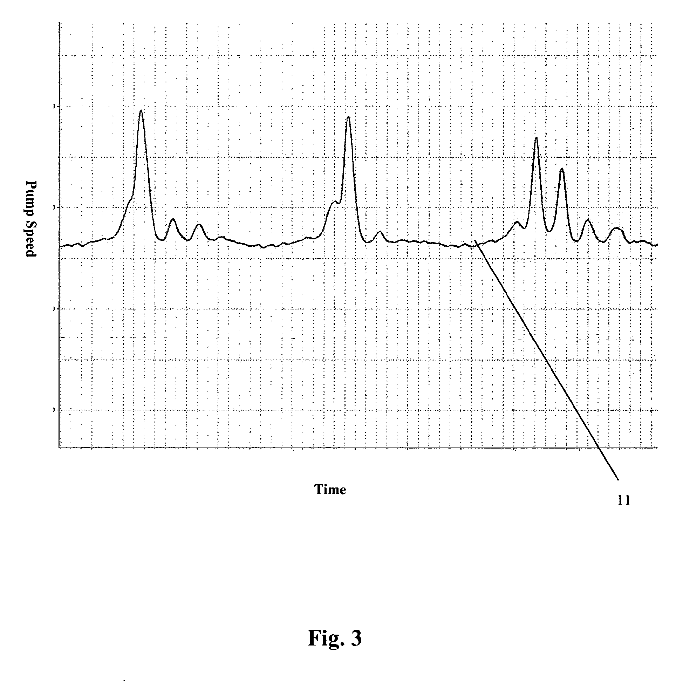

In Fig. 3, a similar plot to Fig. 2 is shown and this graph depicts an additional line 11. The line 11 demonstrates a situation where the patient is experiencing an early stage suction event. Typically, early stage suction events are defined by the left ventricle being pulled across the chamber of the left ventricle towards to intraventricular septum wall at the end of every systole of the cardiac cycle. The pulling of the ventricular wall towards to the septum is caused by the relatively low pressure within the chamber of left ventricle caused by the over-pumping by the rotary blood pump. When the chamber decreases in volume, a majority of the natural pulsatility of the heart is greatly diminished and in the worst cases, the ventricle fully collapses and occludes the inflow cannula of the rotary blood pump which may in turn lead to zero blood flow out of the blood pump and the heart. For example, using the VentrAssist™ blood pump, the pumping speeds under these circumstances generally fluctuate between 2500-2800 rpm. The waveform loses its sinusoidal properties as the pulsatility of the heart drops.

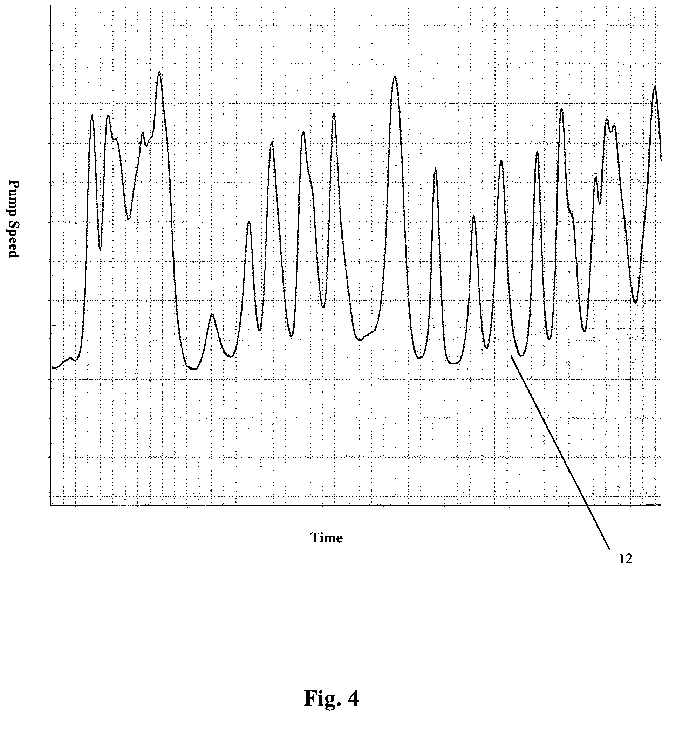

In Fig. 4, a further graph is shown wherein the line 12 depicts the pump speed at a late stage of a suction event. In late stage of ventricular collapse the patient experiences multiple contacts of septum wall with the ventricle wall. For instance, the pump speed of the VentrAssist™ blood pump under these circumstances is generally between 2600 and 3000 rpm. The pumping speed and flow under these circumstances generally becomes erratic and uncontrolled.

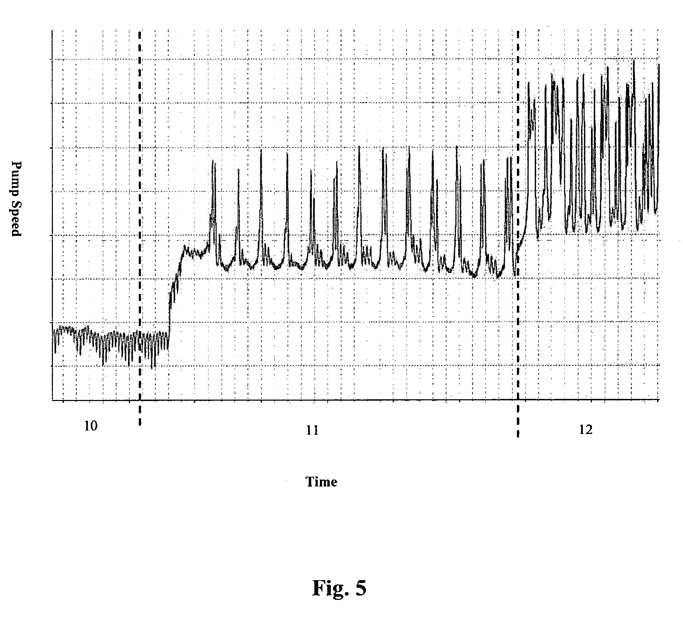

Fig. 5 demonstrates the three states depicted in Figs 2, 3, & 4 combined sequentially into one graph. This graph demonstrates the overall increase in pump speed as the pump struggles with the ventricular collapse of the patient's heart.

The system 1 may also utilise other derivative values of instantaneous speeds including but not limited to: the first, second and third. However, the second derivative is the most preferred.

The preferred system 1 may run continuously in cooperation with other automatic control systems such as systems that automatically control pump speeds in accordance with the physiological needs of the patient.

Also, preferably suction detection system 1 of the first embodiment may be switched on and off by a clinician or doctor, as desired. This switching mechanism may be achieved by the use of a software program that interacts with the pump controller. In some circumstances, it may not be desirable to use an automatic system of suction detection and avoidance. This may occur in situations where the septum wall of left ventricle is particularly weak and early suction events may not be avoided.

A further modification to the above-described systems may be to include different alarms for the different stages of suction events experienced by the patient's heart. For example, the system may include a relatively soft sounding alarm for early stage suction events and relatively loud alarm for late stage events. The system may also preferably log and record the different stages of ventricular collapse and report it to a clinician or doctor at a later time.

In a further embodiment of the present invention, the system 1 additionally includes a system of speed signal filtering. The filtered speed signal may then be used to derive an array of physiological or system parameters. These system parameters may be then used to control the blood pump to avoid ventricular collapse or its imminence.

**Speed Signal Filtering**

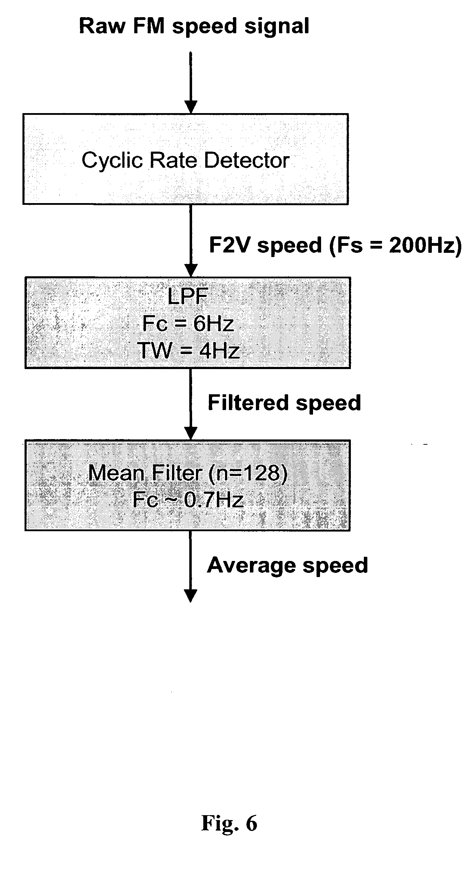

As the pump flow dynamics created by the interaction of the native heart and left ventricle assist device are imprinted onto the non-invasive speed signal, attempts made to identify the pumping states concentrated on waveform analysis of this signal. This analysis was based in large part on considering the relationship between the *filtered* speed and an *average* speed signal: Fig. 6 depicts an embodiment of the process of speed signal filtering which is preferably used in conjunction with the present invention. (Note: in the Figure, TW= Transition Width)

- *F2V* speed: The raw frequency modulated ("FM") speed signal is converted to a frequency output via cyclic rate detection (ie, detecting the positive edge on the FM signal).
- *Filtered* speed: low-pass filter (LPF) with frequency cutoff ($F_{c\_filt}$) = 6 Hz applied to the *F2V* speed signal to remove noise. This data is preferably sampled at $F_s$ = 50Hz for use in subsequent processing.
- *Average* speed: the preferred mean filter of length $128/(200/F_s)$ ($F_{c\_av} \approx 0.7$ Hz) samples applied to the *filtered* signal (or the *F2V* signal if more convenient).

A person skilled in the art may also appreciate that it is possible to alternately use digital filter coefficients and additionally these coefficients may be adjustable through an external programmer including software interfaces.

Preferably, the filters have a constant group delay, so that all frequency components remain equally delayed in time.

**The CART Statistical Method**

*Classification and Regression Tree* (CART) is a binary decision tree algorithm that may be used to predict membership of cases in the classes of a categorical dependent variable from their measurements on one or more predictor variables. CART may be easier to interpret than a multivariate logistic model and considerably more practical to implement in an embedded control system. Furthermore, it is inherently non-parametric, that is, no assumptions are made regarding the underlying distribution of values of the predictor variables. Thus, CART can handle numerical data that is highly skewed or multimodal. Considering the inter-subject variation of the data extracted from the speed signal and the associated skew in its distribution, the CART approach provided the most appropriate method for dealing with the problem of classifying pumping state based on the non-invasive observers.

The speed waveform indices may be used to form the basis for the predictor values used in the CART analysis, while the pumping state provided the categorical dependent variable. In order to determine the optimal time interval over which to classify the data, a series of "window" lengths may be used including, but not limited to 2, 4, 6, 8, 10 and 15 s intervals. Considering the need for a classifier which is both highly accurate and highly responsive (ie, one which requires a minimal amount of time for decision making), a window of 6 s was determined through experimentation to be the most appropriate. However, windows of other sizes may be used including, but not limited to, 2 or 3 seconds.

**Speed Waveform Processing**

Preferably, the filtered and averaged speed signals may be superimposed, then the points at which the filtered speed signal crosses its average from a higher to a lower value are known as "negative crossings", while crossings from a lower to a higher value are "positive crossings". A "speed cycle" will thus be defined as the time interval between successive negative crossings.

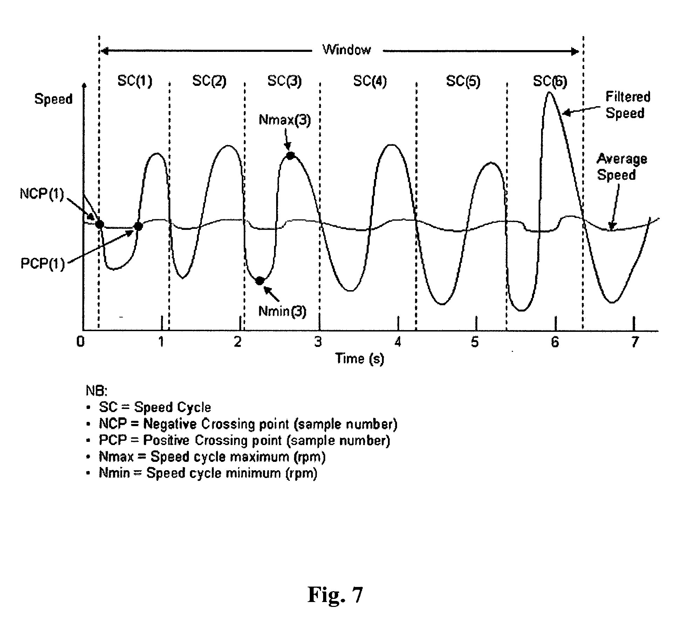

As the data needed to be separated at the junction between speed cycles (since many of the indices are calculated for each speed cycle), the windows were actually taken to end at the first such junction past the stipulated window length (a 6s window length is preferably used). The predictor values were then calculated as either the average or the maximum value of the indices over this window length (indicated for each index below as its "CART predictor"). Fig. 7 depicts an example window in which state detection may be performed.

Speed cycles should preferably exceed a certain number of samples to be processed. In a preferred embodiment, this threshold is based on the requirement that a speed cycle period must exceed an interval equal to twice the highest heart rate: 2*180bpm = 360bpm [= $F_s$/(360/60) = 8 samples ($F_s$ = 50Hz)]. This feature aims to eliminate the error introduced when the average speed signal crosses a relatively jerky filtered speed signal in an undesirable manner. When this requirement is not met, the short interval is considered part of the following speed cycle.

For each index below, its range and applicable preconditions are defined. If after its calculation the index is found to fall outside of its range or fails to obey the precondition, then it should be excluded from further use in determining the associated CART predictor. In the unlikely case where the CART predictor for a certain index (that is actually employed in the current classification tree) cannot be calculated due to range and/or precondition inconsistencies, then state detection cannot be performed for this time period and a suitable note should indicate that this situation was reached.

**Speed Waveform Indices**

The following are the indices or system parameters derived from the speed waveform and at least two said system parameters may form an array of values that may be used to classify pumping state:

1. The speed pulsatility index ($N_{pp}$) generally refers to the difference between the maximum and minimum speed over a speed cycle, indicating the amplitude of the speed signal. This variable is directly related to the contractility of the native heart, with a larger value being associated with a relatively stronger contraction. As pump speed is increased, the native heart imposes relatively less influence on the pulsatility of blood flow through the pump, and $N_{pp}$ will decrease.

$$N_{pp}[i] = N_{max}[i] - N_{min}[i]$$

where:

$N_{max}$ is the maximum speed value for the ith speed cycle
$N_{min}$ is the minimum speed value for the ith speed cycle.

Range: 0-5000rpm
Precondition: $N_{max}[i] > N_{min}[i]$
CART predictor = mean {$N_{pp}[i]$} for all speed cycles in the window.

2. The change in Npp ($\Delta N_{pp}$) (eg, changes in speed pulsatility index) was taken as the difference in consecutive Npp values, signifying the extent to which the speed pulsatility is changing. A high $\Delta N_{pp}$ value is often associated with suction events.

$$\Delta N_{pp}[i] = |\, N_{pp}[i] - N_{pp}[i\text{-}1]\,|$$

Range: 0-5000rpm
CART predictor = mean {$\Delta N_{pp}[i]$} for all speed cycles in the window.

3. The change in $N_{max}$ ($\Delta N_{max}$) was taken as the difference in maximum speed values over consecutive speed cycles. This parameter serves to highlight the changes in speed maxima, where large values may be associated with suction states.

$$\Delta N_{max}[i] = |N_{max}[i] - N_{max}[i\text{-}1]|$$

Range: 0-5000rpm
CART predictor = maximum $\{\Delta N_{max}[i]\}$ for all speed cycles in the window.

4. $N_{profile}$ essentially provides a measure of speed amplitude symmetry or sometimes referred to as an over-pumping index. As the speed waveform demonstrates shorter downward peaks as seen when approaching suction, $N_{profile}$ increases, while the sharp upward peaks evidenced in suction states produce relatively lower $N_{profile}$ values.

$$N_{profile}[i] = (N_{av}[i] - N_{min}[i])/N_{pp}[i]$$

where: $N_{av}[i]$ = average of the filtered speed signal over ith speed cycle.
Range: 0-1
Preconditions: $0 < (N_{av}[i] - N_{min}[i]) < Npp[i]$
CART predictor = mean $\{N_{profile}[i]\}$ for all speed cycles in the window.

5. The change in $N_{profile}$ ($\Delta N_{profile}$) was taken as the difference in consecutive $N_{profile}$ values, thus showing the manner in which the speed profile is changing.

$$\Delta N_{profile}[i] = |N_{profile}[i] - N_{profile}[i\text{-}1]|$$

Range: 0-1
CART predictor = mean $\{\Delta N_{profile}[i]\}$ for all speed cycles in the window.

6. $N_{freq}$ refers to the number of samples between successive crossings of the filtered and averaged speed signal. Each speed cycle will thus have two $N_{freq}$ values associated with it. During the normal pumping state where the speed signal is near-sinusoidal, the $N_{freq}$ values are reasonably constant over time. However, as the waveform becomes less symmetrical, consecutive $N_{freq}$ values will differ from each other. Now, if the change in $N_{freq}$ ($\Delta N_{freq}$) is considered, this parameter will be relatively low for states in which the speed cycle is symmetric about its central crossing point, while higher values will be obtained when such symmetry diminishes.

$$N_{freq}[j] = PCP[(j \text{ div } 2) + 1] - NCP[(j \text{ div } 2) + 1], \text{ for } j \text{ odd}$$

$$= NCP[(j \text{ div } 2) + 1] - PCP[j \text{ div } 2], \text{ for } j \text{ even}$$

$$\Delta N_{freq}[j] = |N_{freq}[j] - N_{freq}[j\text{-}1]|$$

where: j = index of the crossing point interval for this window. (Eg, the 4th crossing point interval of a window is the time period from sample number PCP(2) to NCP(3)).
Range: 0 - 10*$F_s$ samples (ie, maximum of 10s worth of speed samples)
Preconditions: $N_{freq}[j] > 0$
CART predictor = maximum$\{\Delta N_{freq}[j]\}$ for all crossing point intervals in the window. (The size of $\{\Delta N_{freq}[j]\}$ is twice the number of speed cycles for this window)

7. The second time derivative of the speed signal ($\Delta^2 N$) was also noted to be a valuable index of suction detection, due to its ability to recognize sharp peaks in the speed waveform:

$$\Delta^2 N[k] = |\,(N[k] - N[k-1]) - (N[k-1] - N[k-2])\,|$$

where: N[k] = kth sample of the speed signal, preferably sampled at 25Hz
Preferred Range: 0-5000rpm
CART predictor = maximum $\{\Delta^2 N[k]\}$ for all k in the window.

8. There are another two indices that may be used. These are:

$$Nsamp1 = \text{proportion of samples} > (N_{max}[i] + N_{av}[i])/2 \text{ for } i\text{th speed cycle.}$$

$$Nsamp2 = \text{proportion of samples} > (N_{max}[i] + N_{min}[i])/2 \text{ for } i\text{th speed cycle.}$$

where: $N_{av}[i]$ = mean{N[k]} for all k speed samples in the ith speed cycle.

Any combination if these indices may be used. The more indices that are used will generally increase the system reliability and accuracy.

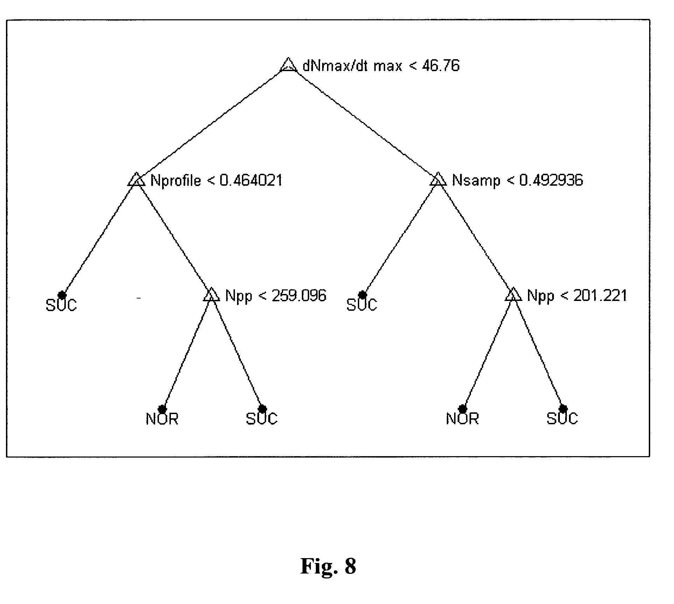

Preferably, a generic tree structure may be provided in an engineering screen of the controller software to be used in conjunction with a left ventricle assist device. The desired tree structure may be defined on a per-patient basis or as required. CART methodologies were used to define various classification trees based on experimental data. Fig. 8 depicts an example of a classification tree based on above-described CART methodology. The indices appearing at each node refer to the CART predictors, while NOR and SUC refer to the normal and suction states respectively.

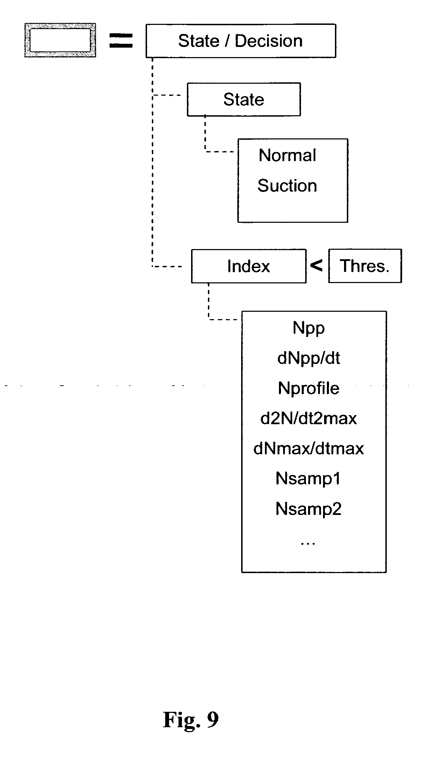

Any of the abovedescribed embodiments may be additionally programmed by software. Such software would provide an interface to allow the controlling conditions to be set or modified. This interface may include, for example, an Engineering Screen that provides an interactive configuration tool for setting Suction Detection parameters. This screen preferably presents a four level binary tree (see Fig. 9). Each of the nodes may be specified as a state or a decision. A set of pull down menus on each node can allow the operator to perform this task including setting the threshold level for each decision node.

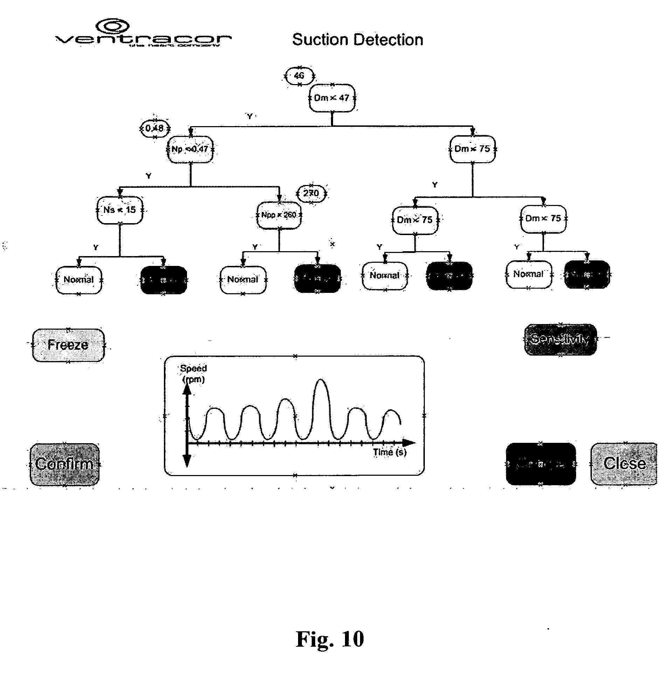

Preferably, if a state is specified at level three or higher in the classification tree there need not be any nodes stemming from this state. In this case, lower nodes would not be visible once a higher node is specified as a state ie, 'Normal' or 'Suction'. In addition, if a decision at level three results in a certain state, the other node automatically assumes the other state. The current speed data block can be displayed as a waveform. Each processed six second block of speed data can result in displayed fired nodes changing colour, including the classification state. In addition, index values can be displayed against each decision node (see Fig. 10).

As soon as a node is modified a prompt can appear on the screen informing the operator that the controller needs to be updated by pressing the 'Update Controller' button before making any assessment of the classification process.

A 'Sensitivity' button can allow the setting of the minimum frequency of suction events that shall trigger suction alarms to be raised on the main screen.

If the controller is updated with the current tree configuration a 'Freeze' button may be provided to stop updating displayed classifications and so giving the operator time to study classifications. If the 'Freeze' button is activated then the 'Update Controller' button need not be visible, no modifications to the tree would then be possible, and the 'Freeze' button would change to an 'Unfreeze' button which reactivates the display updates of each speed data block classification.

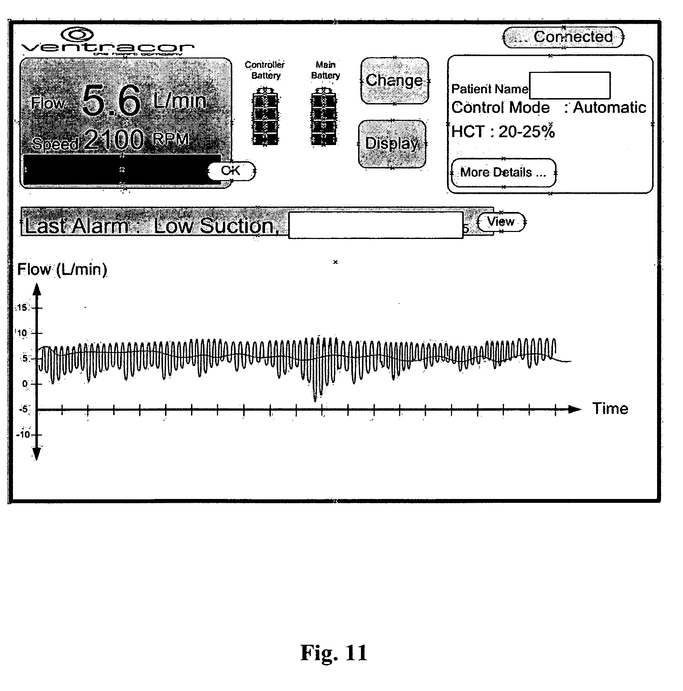

The main screen can comprise a flashing and audible 'Suction' alarm with a displayed measure of their frequency to indicate the level of severity in terms of 'Low', 'Medium' and 'High' classifications (see Fig. 11). This alarm would not be able to be muted or acknowledged. A 'Low Pulsatility' alarm can provide a prophylaxis for suction events. Suction events may also trigger a 'Low Flow' alarm. The 'Suction' alarm shall have priority over the 'Low Flow' alarm.

A window may display an average pump flow trace. The operator may then click or touch a portion of this trace to view the pulsatile waveform. This shall provide a visual means of confirming the existence of suction and its level of severity.

**Examples**

The full potential of Left Ventricle Assist Devices (LVADs) or implantable rotary blood pumps (iRBPs) may be realised through the use of an effective control strategy so that an ambulant patient's metabolic demand for blood flow is met. Ideally, this can be accomplished by providing the ability to discern with accuracy, and preferably avoid, those pumping states which are potentially harmful to the patient. Such states include a collapse of the ventricle due to over-pumping (ventricular suction), or pump back flow (regurgitation) as a result of under-pumping.

The current invention provides an automated approach to the classification of significant pumping states, based on analysis of the non-invasive pump feedback signals. This approach, ideally employing a classification and regression tree (CART), is developed and validated using data obtained from both animal and human recipients of the VentrAssist™ implantable rotary blood pumps (iRBP) (Ventracor Ltd, Chatswood, Sydney, Australia).

*A. Acute Animal Experiments*

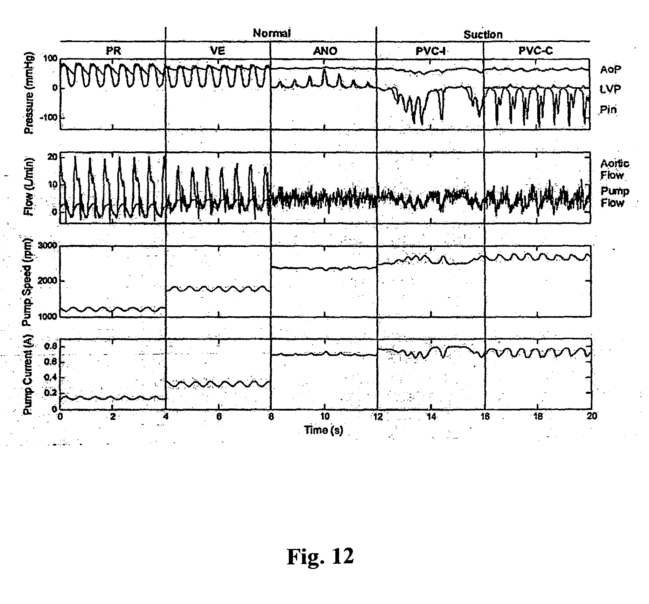

Six healthy pigs were instrumented and implanted with the VentrAssist™ iRBP. In each animal, the pump's inflow cannula was inserted at the apex of the Left Ventricle (LV) while the outflow cannula was anastomosed to the ascending aorta. Various cardiovascular parameters were recorded via invasive measurement (see Fig 12). The non-invasive signals of pump impeller speed, motor current and supply voltage were also recorded for analysis. The transition between pumping states was induced by changes in pump target speed, which was adjusted in variable increments and within variable ranges - depending on the cardiovascular response of each animal - in order to produce the full range of pumping states.

*B. Pumping State Definitions*

Examination of the invasive observers Left Ventricle Pressure (LVP), Aortic Pressure (AoP), aortic flow rate (Qa) and pump flow rate (Qp) indicated the presence of five physiologically significant pumping states: regurgitant pump flow (PR), ventricular ejection (VE), aortic valve non-opening (ANO), and partial collapse (intermittent and continuous) of the ventricle wall during the cardiac cycle (PVC-I and PVC-C). State PR is typified by negative (or regurgitant) Qp during diastole. The most desirable pumping state appeared to be VE, where LV ejection via the aortic valve (AV) occurred in systole and Qp remained positive throughout the cardiac cycle. State ANO occurs when the AV remains closed over the cardiac cycle, and may occur due to decreased myocardial contractility, a relative increase in pump speed, or a decrease in LV preload.

Partial collapse of the left ventricle may be evidenced at relatively high pump speeds, and is commonly characterised by the transient obstruction of the pump inlet cannula as the volume of blood drawn from the LV exceeds that delivered to the heart from the pulmonary circulation. The effects of respiration on cardiac behaviour will often cause partial collapse of the ventricle to occur *intermittently* (state PVC-I), that is, not every heartbeat but over a fraction of the respiratory cycle (when intrathoracic pressure exceeds LVP). State PVC-C maybe exhibited when a suction event occurs every cardiac cycle.

*C. Human Patient Data*

Clinical data was obtained from 10 recipients of the VentrAssist™ iRBP. Surgery and monitoring was performed at The Alfred Hospital (Melbourne, Australia). As for the animal studies, the normal and suction states correlated roughly with pump speed set point. Typically, lower speeds set points exhibited the *normal* pumping state and produced a relatively high level of pulsatility in the speed waveform - residual contractility of the native heart creates an oscillatory flow. As target speed is increased, the influence of the native heart declines and the speed pulsatility is generally reduced. At even higher speeds the *suction* state is induced.

*D. Identifying Pumping States*

In the human patients it was not feasible to measure invasive parameters as in the acute animal experiments. As a result, the non-invasive parameter of speed was itself used to classify the human patient data into three states: normal, suction and equivocal. The equivocal state was assigned to data segments where the pumping state was uncertain, and was excluded in the subsequent analysis. Classification of data into these three states was conducted by examination of the speed waveform by an expert clinician experienced in pumping state identification.

*E. Non-invasive Observers of Flow Dynamics*

Efforts to automate the classification of pumping states focused on waveform analysis of the speed feedback signal. This signal exhibits much of the flow dynamics present within the iRBP, which are in turn affected by the behaviour of the native heart. A number of indices derived from the speed waveform were employed to classify the pumping state, and have been described previously. Briefly, these indices involved features such as amplitude, amplitude symmetry, temporal symmetry and temporal rates of change in various parameters. Analysis of the human patient data employed two additional indices: the proportion of speed samples in a given interval exceeding the midpoint of the maximum and either the minimum or mean speed values.

*F. The CART Statistical Method*

The *Classification and Regression Tree* (CART) method is a binary decision tree algorithm used to predict membership in a number of classes of a categorical dependent variable, based on one or more predictor variables. Given the variability of the indices extracted from the pump speed signal, and the associated skew in its distribution, the CART approach provides an appropriate method for solving the problem at hand. The indices described above formed the basis for the CART predictor variables used in the analysis, while the pumping state provided the categorical dependent variable.

With respect to discerning between normal and suction pumping states, both possible types of misclassification could precipitate clinically significant consequences. While false-negatives (suction events classified as normal) may result in myocardial damage, hemolysis, or lack of perfusion, false-positives may lead to an unnecessary speed reduction by a control system aiming to alleviate the effect of suction. The CART method allowed a symmetrical cost structure to be devised such that the sensitivities of classifying both normal and suction states were comparable.

*G. Treatment of Data*

In the analysis of both the animal and human patient data, data from half the total number of subjects were pooled for use as a training set. The Matlab Statistical Toolbox (The Mathworks, Inc., Natick, Ma, USA) was then employed to build an initial classification tree from this training set. A ten-fold cross-validation was then performed on this set to estimate the true error for trees of various sizes. The optimal tree was determined to be the simplest tree (ie, the tree of smallest size) whose estimated error lay within one standard error of the minimum estimated error. This optimal tree was then validated on the remaining data sets. It should be noted that there was insufficient data corresponding to the PR state to allow inclusion of this state in the analysis.

Performance of the state detection method was assessed by a comparison of the state ascertained by the optimal tree and the 'known' state determined via invasive measurement (for the animal experiments) or via expert opinion (for the human patients). The sensitivity and specificity associated with each state were used to quantify the system's performance. After analysing a range of window lengths, a length of 6s was deemed most suitable, considering the need to balance the trade off between accuracy and resolution.

Results based on data from the acute animal experiments indicate the high level of accuracy achieved in correctly identifying most pumping states. Perhaps the only questionable state in this regard, was PVC-C, with a sensitivity of 61.2%. However, when considered together with PVC-I as one *suction* state, the sensitivity increases to 100%, indicating that the lack of accuracy was due to misclassification between these two suction states (rather than the normal states). When a simplified binary scheme is evaluated (ie, when only two initial states, *suction* and *normal,* are considered), sensitivities of 100% were attained for both the *normal* and *suction* states.

Variability between patients, and even within a single patient over time, presents a significant challenge to the development of robust pumping state detection algorithms. Individuals suffering heart failure typically exhibit a wide range of severity in their condition, often with unique cardiovascular characteristics such as: residual contractility of the ventricle, systemic resistance, and blood pressure level. These characteristics ultimately determine the nature of the interaction between the iRBP and the patient's native heart. As a result of this interaction and the inherent physiological variability, any indices derived from the non-invasive pump signals exhibit a concomitant level of variability in both their temporal dynamics and their global statistics. Any automated pumping state classification system must perform in the face of this variability and preferably, without the need for patient-specific calibration.

In this regard, the classification scheme therein described is particularly encouraging, achieving a high level of accuracy when classifying pumping states for both the animal and clinical data. This success is likely due to the use of combination of speed-derived indices, and their integration into the CART model. It is also interesting to note that those indices were considered independently of the patient. As a result, the classifiers developed purport to be extremely robust, obviating the need for a patient-specific calibration procedure to account for physiological variation.

There exists an inherent difference in the behaviour of the native heart when comparing animal and human subjects. While the animals possess relatively healthy cardiac function, human implant recipients are suffering with a failed left ventricle (due to hypertension, cardiomyopathy, coronary artery disease, or a range of other causes). The ventricular dynamics exhibited by patients may be likely to produce pump signal waveforms with different features to the healthy animal subjects, and in turn will influence the non-invasive indices upon which the classification system is based. In light of this, the approach to pumping state classification described here - employing multiple indices integrated into a CART model - appears to work very well.

Comparing the results for detecting *suction* versus *normal* states, we see an excellent performance when applied to data from the acute animal experiments. When applied to data from the human clinical trial, the classifier achieves only 99.17% sensitivity and 98.29% specificity. This is perhaps largely due to the wide variation in cardiac condition and dynamics observed in the human patients, and the consequent disparity in suction detection indices. Furthermore, there was a larger corpus of data available relating to the human trials (10 patients; 13 048 records) than was available for the acute animal studies (6 animals; 388 records). Nevertheless, the performance of the classifier, even in the face of significant variability inherent in the data from human patients, is impressive, testifying to the suitability of the approach and the robustness of the resulting algorithms.

The above descriptions detail only some of the embodiments of the present invention. Modifications may be obvious to those skilled in the art and may be made without departing from the scope and spirit of the present invention.

## Claims

1. A control system for controlling the target speed of a rotary blood pump, said control system being adapted to measure the speed and/or power of said pump, and then to calculate at least two system parameters derived from said speed and/or power measurement, and to analyse said parameters, and if said analysis indicate ventricular collapse or imminent ventricular collapse, then said control system alters the target speed of said pump to minimise the risk of said collapse occurring.

2. The control system of claim 1, wherein said system parameters include calculating the pulsatility index ($N_{pp}$).

3. The control system of claim 1, wherein said system parameters include calculating the change in the pulsatility index ($\Delta N_{pp}$).

4. The control system of claim 1, wherein said system parameters include calculating the second time derivative of the speed ($\Delta^2 N$).

5. The control system of claim 1, wherein said system parameters include calculating the $N_{profile}$.

6. The control system of claim 1, wherein said system parameters include calculating the change in $N_{profile}$ ($\Delta N_{profile}$).

7. The control system of claim 1, wherein said system parameters include calculating the change in $N_{max}$ ($\Delta N_{max}$).

8. The control system of claim 1, wherein said system parameters include calculating the change in $N_{freq}$ ($\Delta N_{freq}$).

9. The control system of claim 1, wherein said system alters the target speed by reducing it to a predetermined target speed.

10. The control system of claim 1, which uses Classification and Regression Tree (CART) analysis to analyse said parameters.

11. The control system of claim 1, wherein said pump is an implantable pump.

12. A control system for controlling the target speed of a rotary blood pump, said control system being adapted to measure the speed and/or power of said pump, and then to calculate one or more system parameters derived from said speed and/or power measurement, and then to analyse said parameters, using a Classification and Regression Tree (CART) analysis, and if said analysis indicates ventricular collapse or imminence of ventricular collapse, then said control system alters the target speed of said pump to minimise the risk of said collapse occurring.

13. The control system of claim 12, wherein said system parameters are selected from among a calculation of: the pulsatility index ($N_{pp}$), the change in the pulsatility index ($\Delta N_{pp}$), the second derivative of the speed ($\Delta^2 N$), the $N_{profile}$,

the change in $N_{profile}$ ($\Delta N_{profile}$), the change in $N_{max}$ ($\Delta N_{max}$), or the change in $N_{freq}$($\Delta N_{freq}$).

14. An apparatus for controlling the target speed of a rotary blood pump, said apparatus having means to measure the speed and/or power of said pump, and means to calculate at least two system parameters derived from said speed and/or power measurement, and means to analyse said parameters, and if the analysis indicates ventricular collapse or imminent ventricular collapse, then said apparatus alters the target speed of said pump to minimise the risk of said collapse occurring.

15. The apparatus of claim 14, wherein said system parameters are selected from among a calculation of: the pulsatility index ($N_{pp}$), the change in the pulsatility index ($\Delta N_{pp}$), the second derivative of the speed ($\Delta^2 N$), the $N_{profile}$, the change in $N_{profile}$ ($\Delta N_{profile}$), the change in $N_{max}$ ($\Delta N_{max}$), or the change in $N_{freq}$($\Delta N_{freq}$).

16. The apparatus of claim 14, which uses Classification and Regression Tree (CART) analysis to analyse said parameters.

17. A method of controlling the target speed of a rotary blood pump, said method comprising, measuring of the speed and/or power of said pump, calculating at least two system parameters derived from the speed and/or power measurement, analysing said parameters, and if said analysis indicates ventricular collapse or imminent ventricular collapse, then altering the target speed of said pump so as to minimise the risk of said collapse occurring.

18. The method of claim 17, wherein said system parameters are selected from among a calculation of: the pulsatility index ($N_{pp}$), the change in the pulsatility index ($\Delta N_{pp}$), the second derivative of the speed ($\Delta^2 N$), the $N_{profile}$, the change in $N_{profile}$ ($\Delta N_{profile}$), the change in $N_{max}$ ($\Delta N_{max}$), or the change in $N_{freq}$($\Delta N_{freq}$).

19. The method of claim 17, which uses Classification and Regression Tree (CART) analysis to analyse said parameters.

Measure Back EMF generated by the blood pump — 2

Calculate estimated instantaneous speed from back EMF — 3

Calculate the second derivative of estimated speed over a predetermined time internal — 4

Pump controller manually reset by doctor or clinician — 9

Is $2^{nd}$ derivative of speed above threshold value (i.e. is suction detected?) — 5

No

Yes

1

Reduce pump speed to run a predetermined default speed — 6

Log suction event — 7

Activate alarm — 8

**Fig. 1**

**Fig. 2**

**Fig. 3**

**Pump Speed**

**Time**

12

**Fig. 4**

**Fig. 5**

**Raw FM speed signal**

Fig. 6

Fig. 7

**Fig. 8**

State / Decision

State

Normal
Suction

Index $<$ Thres.

Npp

dNpp/dt

Nprofile

d2N/dt2max

dNmax/dtmax

Nsamp1

Nsamp2

...

**Fig. 9**

**Fig. 10**

**Fig. 11**

**Fig. 12**

**Fig. 13**

**European Patent Office**

## PARTIAL EUROPEAN SEARCH REPORT

which under Rule 45 of the European Patent Convention shall be considered, for the purposes of subsequent proceedings, as the European search report

Application Number

EP 07 25 1636

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 01/72352 A9 (CLEVELAND CLINIC FOUNDATION [US]; MEDVEDEV ALEXANDER [US]; GOLDING LEO) 19 December 2002 (2002-12-19)<br><br>* figure 8 *<br>* page 7, line 13 - page 8, line 11 *<br>* page 8, lines 24-26 *<br>* page 9, line 11 - page 10, line 25 *<br>* page 11, lines 9-14 *<br>* page 11, line 32 - page 12, line 34 *<br>* page 13, lines 24-27 *<br>----- | 1,2,11,<br>14,15<br><br>3,4 | INV.<br>A61M1/12<br>A61M1/10 |
| D,X | WO 2004/028593 A (VENTRASSIST PTY LTD [AU]; AYRE PETER JOSEPH [AU]) 8 April 2004 (2004-04-08)<br>* figures 6,7,9 *<br>* page 12, lines 5-23 *<br>* page 15, line 16 - page 16, line 1 *<br>* page 16, line 23 - page 18, line 25 *<br>* page 20, lines 5-9 *<br>* page 21, line 20 - page 22, line 17 *<br>* page 23, line 1 - page 25, line 23 *<br>-----<br>-/-- | 1,2,9,<br>11,14,15 | |

**TECHNICAL FIELDS SEARCHED (IPC)**

A61M

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 6 August 2007 | Hochrein, Marion |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

**PARTIAL EUROPEAN SEARCH REPORT**

Application Number

EP 07 25 1636

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| D,X | US 6 991 595 B2 (BURKE DAVID J [US] ET AL) 31 January 2006 (2006-01-31) * figures 1,2 * * column 3, line 47 - column 4, line 11 * * column 4, lines 31-37 * * column 4, line 55 - column 5, line 8 * * column 5, lines 20-29 * * column 5, lines 47-53 * * column 6, lines 4-29 * * column 6, line 38 - column 7, line 15 * ----- | 1,2,9, 11,14,15 | |
| D,Y | WO 03/057013 A (MICROMED TECHNOLOGY INC [US]; MORELLO GINO F [US]) 17 July 2003 (2003-07-17) * page 6, lines 17-24 * * page 7, lines 1-12 * * page 7, lines 18-25 * * page 8, lines 7-29 * * page 12, line 32 - page 13, line 14 * * figures 8,9 * ----- | 4 | TECHNICAL FIELDS SEARCHED (IPC) |
| D,Y | US 6 066 086 A (ANTAKI JAMES F [US] ET AL) 23 May 2000 (2000-05-23) * figures 4-6,11,12 * * column 4, line 62 - column 5, line 3 * * column 5, lines 34-67 * * column 6, line 57 - column 7, line 21 * ----- | 3 | |
| X | VOLLKRON M ET AL: "Development of a Suction Detection System for Axial Blood Pumps" ARTIFICIAL ORGANS, BLACKWELL SCIENTIFIC PUBLICATIONS, INC., BOSTON, US, vol. 28, no. 8, August 2004 (2004-08), pages 709-716, XP009087840 ISSN: 0160-564X * abstract; figures 1,2 * ----- | 1,14 | |

**European Patent Office**

**INCOMPLETE SEARCH SHEET C**

Application Number

EP 07 25 1636

Claim(s) not searched:
    17-19

Reason for the limitation of the search (non-patentable invention(s)):

Article 52 (4) EPC - Method for treatment of the human or animal body by therapy

## ANNEX TO THE EUROPEAN SEARCH REPORT
## ON EUROPEAN PATENT APPLICATION NO.

EP 07 25 1636

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-2007

| Patent document cited in search report | | | Publication date | Patent family member(s) | | | Publication date |
|---|---|---|---|---|---|---|---|
| WO 0172352 | A9 | | 19-12-2002 | AT | 283077 | T | 15-12-2004 |
| | | | | AU | 4757301 | A | 08-10-2001 |
| | | | | CA | 2404636 | A1 | 04-10-2001 |
| | | | | CZ | 20023242 | A3 | 15-01-2003 |
| | | | | DE | 60107401 | D1 | 30-12-2004 |
| | | | | DE | 60107401 | T2 | 24-11-2005 |
| | | | | EP | 1267958 | A2 | 02-01-2003 |
| | | | | JP | 2004501678 | T | 22-01-2004 |
| | | | | MX | PA02009410 | A | 17-05-2004 |
| | | | | WO | 0172352 | A2 | 04-10-2001 |
| WO 2004028593 | A | | 08-04-2004 | CA | 2500554 | A1 | 08-04-2004 |
| | | | | EP | 1549363 | A1 | 06-07-2005 |
| | | | | JP | 2006500982 | T | 12-01-2006 |
| US 6991595 | B2 | | 31-01-2006 | AT | 329636 | T | 15-07-2006 |
| | | | | DE | 60306012 | T2 | 11-01-2007 |
| | | | | EP | 1354606 | A1 | 22-10-2003 |
| | | | | ES | 2268202 | T3 | 16-03-2007 |
| | | | | US | 2003199727 | A1 | 23-10-2003 |
| WO 03057013 | A | | 17-07-2003 | AU | 2003202250 | A1 | 24-07-2003 |
| | | | | CA | 2471484 | A1 | 17-07-2003 |
| | | | | CN | 1638824 | A | 13-07-2005 |
| | | | | EP | 1469770 | A2 | 27-10-2004 |
| | | | | JP | 2005514094 | T | 19-05-2005 |
| | | | | US | 2005004418 | A1 | 06-01-2005 |
| US 6066086 | A | | 23-05-2000 | AT | 299721 | T | 15-08-2005 |
| | | | | AU | 713592 | B2 | 09-12-1999 |
| | | | | AU | 4963197 | A | 29-05-1998 |
| | | | | CA | 2241888 | A1 | 14-05-1998 |
| | | | | DE | 69733746 | D1 | 25-08-2005 |
| | | | | DE | 69733746 | T2 | 08-06-2006 |
| | | | | EP | 0877633 | A2 | 18-11-1998 |
| | | | | ES | 2243987 | T3 | 01-12-2005 |
| | | | | WO | 9819624 | A2 | 14-05-1998 |
| | | | | JP | 2000504977 | T | 25-04-2000 |
| | | | | US | 5888242 | A | 30-03-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6227797 B, Watterson
- US 6949066 B, Bearnson
- US 6991595 B, Burke
- US 6783328 B, Lucke
- US 5888242 A, Antaki
- US 6066086 A, Antaki
- US 6623420 B, Reich
- US 6443983 B, Nagyszalancy
- WO 03057013 A, Morello
- WO 04028593 A, Ayre